# EUROPEAN PATENT SPECIFICATION

(11) **EP 1 763 359 B1**
(45) Date of publication and mention of the grant of the patent: **08.10.2014**
(21) Application number: 05742921.9
(22) Date of filing: 27.04.2005
(51) Int. Cl.: A61K 36/27, A61P 3/04, A61K 36/752, A61K 36/82, A61K 36/185, A61K 36/328, A61K 36/38, A61K 36/42, A61K 36/48, A61K 36/484, A61K 36/54, A61K 36/899

(54) **USE OF PREGNANE GLYCOSIDES IN THE TREATMENT/MANAGEMENT OF OBESITY**
VERWENDUNG VON PREGNAN-GLYKOSIDEN BEI DER BEHANDLUNG/KONTROLLE VON FETTSUCHT
UTILISATION DE GLUCOSIDES DE PREGNANE POUR LE TRAITEMENT /GESTION DE L'OBESITE

(30) Priority: 27.04.2004 IN CH03842004
(43) Date of publication of application: 21.03.2007
(73) Proprietor: Rajendran, Ramaswamy, Bangalore 560 071 (IN); Rajendran, Kamala, Bangalore 560 071 (IN)
(72) Inventor: Rajendran, Ramaswamy, Bangalore 560 071 (IN); Rajendran, Kamala, Bangalore 560 071 (IN)
(74) Representative: Demski, Siegfried
(86) International application number: PCT/IN2005/000131
(87) International publication number: WO 2005/102371

(56) References cited:
- RIZWANI G H ET AL: "BIOLOGICAL EFFICACY OF THE EXTRACTS AND CONSTITUENTS OF CARALLUMA TUBERCULATA AND C. EDULIS" JOURNAL OF FACULTY OF PHARMACY OF GAZI UNIVERSITY, GAZI UNIVERSITY, ANKARA, TR, vol. 11, no. 1, 1994, pages 43-53, XP009036980 ISSN: 1015-9592
- AHMAD V U ET AL: "NEW PREGNANE GLYCOSIDES FROM CARALLUMA-TUBERCULATA" JOURNAL OF NATURAL PRODUCTS, vol. 51, no. 6, November 1988 (1988-11), pages 1092-1097, XP009036962 ISSN: 0163-3864
- RAMESH M ET AL: "ANTINOCICEPTIVE AND ANTI-INFLAMMATORY ACTIVITY OF CARUMBELLOSIDE-I ISOLATED FROM CARALLUMA UMBELLATA" 15 December 1999 (1999-12-15), JOURNAL OF ETHNOPHARMACOLOGY, ELSEVIER SCIENTIFIC PUBLISHERS LTD, IE, PAGE(S) 349-352 , XP001183713 ISSN: 0378-8741 the whole document

## Description

This invention relates to pregnane glycosides in the form of extracts of the caralluma species of plants for use in the treatment of obesity.

Obesity is a major public health problem the world over in view of the direct and indirect economic and social costs of obesity. One of the major causes of obesity is the stressful and sedentary lifestyles of modern life and the widespread adoption of diets that contain large amounts of high calorie processed foods. The problem is particularly acute and widespread in some industrialized countries.

Obesity is a direct causal contributor to the pathophysiology of a number of diseases and causes exacerbation in several others. Some of said disorders/symptoms are diabetes, hypertension, cardio-vascular disease, atherosclerosis, stroke and others.

Obesity is being increasingly combated medically by providing treatments for weight-reduction and for coping with, and management of associated symptoms/disorders such as high blood sugar, b.p., joint pains and others.

Weight reduction and related treatments such as regulation of BMI (Body Mass Index); increasing lean mass, increasing BMR (Basal Metabolic Rate), are also being increasingly adopted by people who are not strictly clinically obese but do so for personal or social reasons such as the desire to feel and look good. The use/methods/compositions of the invention are also relevant to said people.

The document of "Biological efficacy of the extracts and constituents of Caralluma tuberculata and C. Edulis", Rizwani et al., Journal of Faculty of Pharmacy of Gazi University, vol. 11, no. 1, 1994, pages 43-53, XP009036980 discloses the biological activity of the ethanolic and aqueous extracts of both plants. The hypoglycaemic activity of extracts of Caralluma tuberculata has been determined with rats.

The blood glucose levels were estimated by the method of Nelson and Somogyi after administration of aqueous and ethanolic extracts dissolved in water. According to this document extracts of caralluma tuberculeta have a blood sugar lowering effect.

The document "Antinociceptive and anti-inflammatory activity of carumbelloside-I isolated from Caralluma umbellata", Ramesh et al., Journal of Ethnopharmacology, 1999-12-15, vol. 68, 349-352, XP001183713 discloses that a special pregnane glycoside named carumbelloside-I isolated from caralluma umbellata has an antinociceptive activity and no anti-inflammatory activity.

The document "New pregnane glycosides from Caralluma tuberculata", Ahmad et al., Journal of Natural Products, vol. 51, No. 6, 1988-11, 1092-1097, XP009036962 discloses a study in which the molecule structure of two special pregnane glycosides, named caratuberside A and B, were determined.

The problem relating to the state of the art is to establish a drug for the treatment of obesity, which exhibits no or small toxicity and hazardous side effects.

The problem is solved by pregnane glycosides in the form of extracts of the caralluma species of plants for use in the treatment of obesity, wherein said treatment comprises the administration of a daily main dose thereof to a subject over a period of time of 3 to 4 month, the pregnane glycoside content of said main dose being specified by the molecularly equivalent amount of caratuberside therein and is from 250 mg to 500 mg caratuberside in case the BMI of the subject is from 25 to 30 or 500 mg to 1000 mg caratuberside in case the BMI of the subject is from 30 to 50.

It is the observation of these inventors that the pregnane glycosides and other constituents of caralluma are highly effective for treatment and management of obesity and obesity-related disorders/symptoms and other said disorders. Said glycosides have been tested to be non-toxic and generally free of side effects. In cases where side effects were observed during the tests they were only gastro-intestinal (GI) in nature and were minimal and transient. Said side effects were generally found to cause within about a week of the commencement of treatment.

The principals of caralluma relevant to said uses of this invention are said pregnane glycosides thereof. Preferably, said glycosides are caratuberside and/or bouceroside or mixtures thereof and includes the isomers thereof. More preferably, said compositions are mixtures of caratuberside and bouceroside which are found to exhibit strong synergy effects particularly with respect to their applications for use in the treatment and management of obesity.

Preferably, the ratio of caratuberside and bouceroside in compositions for said uses and said methods of the invention is from 9:1 to 19:1 by wt. Some compositions of this invention further comprise the saponin glycoside(s) and the bitters of caralluma and/or other additional therapeutical, nutraceutical or nutritional components.

The pregnane glycosides for said uses may be isolated from materials of the caralluma species of plants. Or they may be one of the extracts of the caralluma species of plants. As the caratuberside-bouceroside ratio (CBR) found in nature in the caralluma species of plants is almost equal to the value at which said synergy is a maximum it is advantageous to directly employ a caralluma plant extract in said uses of the invention. These inventors have developed said caralluma plant extracts and processes of making the same by the extraction of caralluma plant matter. This forms the subject-matter of their application for Indian patent No. 451/MAS/2003 dated 4th June 2003.

Thus, this invention provides for said treatment and management and said alteration/improvement/ regulation by the use of, that is, administration of said pregnane glycosides, preferably caratuberside and bouceroside. This invention, therefore, focuses on said caratuberside-bouceroside mixtures that offer considerable synergy and in particular, mixtures having caratuberside content from about 90% to about 95% w/w. for said uses, methods and compositions of the invention.

Caralluma plants also contain some saponin glycoside compounds which are present in small quantities and which are precursors of a number of useful products. However, their role in said uses and methods of the invention is insignificant except for one application discussed hereinbelow and could therefore be left substantially unextracted when preparing said caralluma pregnane glycoside extracts for the uses/ methods/compositions of the invention. The term 'glycoside(s)' in the further specification hereinbelow, therefore, generally refers to said pregnane glycosides unless otherwise required by the context.

Caralluma plant matter also contains bitters which are also of significance medically and healthwise and some novel applications thereof are provided hereinbelow wherein said pregnane glycoside(s) includes some amounts of said saponin glycoside(s) and/or said bitters.

It would be possible to isolate any of the caralluma glycosides in pure form and then use them either singly or as mixtures in the said uses of the invention. Alternatively, said glycosides could be obtained by synthesis methods. A still further alternative is to obtain a mixture of caratuberside and bouceroside by extraction of caralluma plant matter(or any other plant matter commining pregnane glycosides). References to glycosides also mean reference to their isomers. Two isomers of caratuberside and ten of bouceroside are known to be present in caralluma. Within the scope of the invention, said glycosides in the uses/methods/compositions of the invention may be in their unconverted forms or in the form of any of the pharmaceutically accepted salts thereof. Any of the pharmaceutically acceptable carriers may be used with the glycosides which may be in their converted or unconverted forms.

As mentioned, this invention preferably provides for the use of the caralluma extracts wherein by carefully conducting the extraction process substantially the entire caratuberside and bouceroside content in the plant material is extracted and the said CBR value in the plant material substantially preserved in the extract. The said earlier application for patent by these inventors provides both a liquid form extract of glycosides in aq. ethanol solution and solid form extract wherein the extracted glycosides are adsorbed on a suitable excipient. These extracts are easily convertible into any of the pharmaceutically acceptable forms for administration to subjects, such as for example, tablets, capsules, suspensions and injections. Conversion of the glycosides in the extracts into the form of any of the pharmaceutically acceptable salts may also be easily carried out if required and said salts are within the scope of the invention. Said conversions may be carried out by any of the known processes.

Known pharmaceutical options for treatment of obesity, that is, for weight reduction are: thermogenesis, lipase inhibitors and compounds that suppress appetite and/or stimulate the central nervous system(CNS).

The thermogenesis method involves the raising of the body core temperature slightly. This increases the metabolism of deposited lipids in the body. Thermogenesis drugs act on the brain and the thyroid gland resulting in said increase of body core temperature.

Lipase inhibitors work by reducing absorption of fat in the intestine system. Thus, when a lipase inhibitor is administered to a subject, the fat portion of the food consumed by the subject passes through his intestinal system unabsorbed and is eliminated in the stools.

Appetite suppressants/CNS stimulators act by modifying the levels of neurotransmitters such as catecholamine and serotonin in the blood leading to decreased feeling of hunger.

All three abovementioned approaches to obesity treatment and management have been found to have strong to unacceptable side effects.

Thermogenesis, by its nature, carries risk of side effects such as overstimulation of vital functions such as cardiac rhythm, blood pressure, neurotransmitter levels and the endocrine system. Subjects experience nervousness, anxiety, hypersensitivity to stimulii, insomnia and irregular heartbeats.

The side effects associated with known lipase inhibitors are GI in nature. Patients report oily and fatty stools and increased bowel movement. They also complain of urgency of bowel movement and sometimes inability to control the same. Oily spotting may also occur between bowel movements. A yet another side effect is the loss of fat soluble vitamins present in the food. They are carried away by the unabsorbed fat into the stools. For these reasons, patient compliance is found to be a problem in lipase inhibitor treatments.

When appetite suppressants and CNS stimulators are used, side effects arise from the altered neurotransmitter function. These include increased heart rate, hypertension, anxiety, mood alterations, diaphoresis, dizziness, swelling of extremities, dryness of mouth, constipation and insomnia.

Said known obesity treatments are, furthermore, contraindicated in many clinical situations such as hypertensive obese patients or patients suffering from coronary artery disease, cardiomegaly and some chronic GI disorders such as Irritable Bowel Syndrome.

In contrast, as observed by these inventors, pregnane glycosides act without interfering with the digestion process or thermogenesis or neurotransmitter levels. A caralluma extract(containing the pregnane glycosides thereof) acts on the Kreb's cycle(citric acid cycle) at critical points thereof such as to inhibit fat synthesis in the liver and other cells of the subject and enhance fat burning(fat metabolism).

These inventors believe that pregnane glycosides also act on the hunger centres in the brain and reduce the feeling of appetite. Remarkably, this action occurs in the case of pregnane glycosides without any side effects arising, as was first observed by these inventors. Pregnane glycosides seem to suppress appetite without causing any significant disturbance in the neurotransmitter functioning unlike as in the case of the known appetite-suppressants. These inventors have further found that pregnane glycosides also apparently increase thermogenesis without manifestation of any side effects observed with the known thermogenesis enhancers.

Caralluma is indeed a food item that has been consumed in the past, and still consumed as a food by some population groups in India. Its non-toxicity, and that of the pregnane glycosides thereof, is therefore well established and further verified by clinical tests conducted by these inventors. This invention, therefore, provides for caralluma pregnane glycosides(and said extracts) not only as a medicinal product but also as a health-giving and health-ensuring food supplement. Thus, this invention offers caralluma pregnane glycosides and other caralluma constituents also as nutraceutical products for consumption either by themselves or in conjunction or mixture with a variety of other food supplements, energy enhancers and other health products.

Caralluma is a group of succulent species found wild in India, Afghanistan, Arabia, Southern Europe, Sri Lanka and others. Caralluma plants are small, erect and fleshy although some species have recumbent stems. The stems are 4-grooved and are from 10-40 mm thick(1/2" to 1-1/2"). The spines of the stems are actually the leaves of the plant. Caralluma plants bear star-shaped, fleshy flowers in a range of dark colours from purple and black to red and dark brown. Well over two hundred caralluma species are known. Some of the caralluma species investigated by these inventors are: C. indica, C. fimbriata, C. attenuata, C. tuberculata, C,. edulis, C. adscendens, C. stalagmifera, C. umbellata, C. lasiantha and C. penicillata. Although some limited and non-conclusive tests and animal trials appear to have been done in the past on some medicinal properties of caralluma, no tests, animal trials or human clinical trials appear to have been conducted on the appetite-suppression property or on weight reduction and other obesity symptoms related properties of pregnane glycosides. This also applies to the properties of said glycosides related to the ether disorders/symptoms mentioned hereinabove.

This invention therefore, is apparently first to establish the appetite-suppression, weight reduction and related properties of caralluma extracts relevant to treatment and management of obesity and also the properties relevant to said alteration/regulation/improvement of said conditions/parameters/functions. The properties have been established by carefully designed and conducted animal trials and subsequently clinical trials on human subjects. This invention is also first in correlating said properties to the pregnane glycosides contained in the caralluma species of plants and to other members of the pregnane glycosides group.

Thus, this invention provides for novel uses of caralluma glycosides and/or said caralluma extracts in the treatment and management of obesity wherein said glycosides and/or extracts are administered to achieve weight reduction, reduction of BMI, reduction of fat, reduction in waist, hip and arm circumferences, reduction of blood glucose, reduction of blood pressure, increase of lean body mass, increase of BMR, reduction in blood cholesterol, enhancement of the blood HDL/LDL ratio, appetite suppression, enhanced stamina, energy and endurance levels, improved hearing, improved capillary health, improved cognitive and memory function and/or for treatment and management of clinical depression, migraine, osteo-arthritis, aging syndrome, menopausal syndrome, mood elevation and joint inflammation and to improve/regulate these parameters/conditions/functions.

This invention has found that per day dosage of between 10 mg. to 1500 mg. of caratuberside or caratuberside-bouceroside mixtures do not exhibit any toxicity or side effects except for said transient effects experienced by some subjects. In the clinical trials that are described further hereinbelow the dosage followed was 300 mg. per day of said caratuberside-bouceroside mixture, the ratio of the two said components(CB Ratio) therein being from about 9:1 to about 19:1 by wt. Subsequently higher doses of about 450 mg. per day per subject were adopted. Increased doses resulted correspondingly in increased change in the parameter(s), all other things being equal indicating that the two were generally proportional. These inventors observe that this proportionality of doses and effects extends upto at least 1500 mg. per day doses. Thus, this invention provides for designing the said doses to obtain desired speeds of transformation of said parameters/functions/conditions.

Said doses may contain said glycosides in the unconverted forms or otherwise. Said glycosides, converted or unconverted may be associated with any of the known pharmaceutically accepted carriers and excipients and furthermore be in the form of any of the pharmaceutically accepted salts. The compositions may include any pharmaceutically acceptable and/or edible colouring agents, flavouring agents and other additives.

The biochemical processes of carbohydrate, protein and fat metabolism and off the breakdown and biosynthesis of fats relevant are summarised below.
i. Carbohydrates, proteins and fats are broken down in cells to generate energy in the form of energy carrying molecules such as ATP(Adenosme triphosphate). Said breakdown also produces pyruvic acid that diffuses into the mitochondria where a series of reactions produces, *inter alia,* acetyl coenzyme A and oxaloacetate. Compounds NAD and FAD are also produced. They carry activated hydrogen atoms that subsequently take part in fat synthesis reactions. A further reaction links acetyl coenzyme A and oxaloacetate to give a molecule that is capable of diffusion across the mitochondrial wall into the cell cytoplasm. The NAD and FAD also diffuse out into the cytoplasm where together with acetyl coenzyme A they undergo various reactions ending with synthesis of fat molecules.
ii. In the cytoplasm an enzyme called citrate lyase catalyses the breakdown of said combined molecule into its constituent parts, oxaloacetate and acetyl coenzyme A. The action of citrate lyase is critical in that blocking the action thereof would prevent formation of acetyl coenzyme A in the cytoplasm and thereby disrupt the fat synthesis process in the cells.
iii. The precursor(building block) of fat synthesis in the cells is malonyl coenzyme A which is produced from acetyl coenzyme A. Malonyl coenzyme A is the key to fat synthesis in cells and if the production thereof is either prevented or restricted, the fat synthesis is similarly affected.
iv. Both fat breakdown(fat metabolism) and fat synthesis occur simultaneously in cell cytoplasm particularly in liver cells. The fat breakdown is promoted(catalysed) by an enzyme called carnitine acyltransferase. Relative levels of carnitine acyltransferase and malonyl coenzyme A determine the balance between the twin reactions of fat synthesis and breakdown, more of one promoting one while more of the other promoting the other.
v. Acetyl coenzyme A is also consumed in the mitochondria to generate/release energy. Only when the energy requirements of a cell are met that excess acetyl coenzyme A gets formed that migrates to the cytoplasm where it partakes in fat synthesis as mentioned above.
vi. An important factor is the feeling of satiety/hunger that arises in the hypothalamus. The hypothalamus receives signals from the stomach conveying the position, that is, the fullness or otherwise of the stomach. This is translated into the appropriate feeling of hunger or satiety in the brain. Via another channel the brain also receives signals indicating the position about glucose and glycogen levels in the liver. If these levels are high, they generate a feeling of satiety in the brain and vice versa.

This invention observes that pregnane glycosides block the action of said enzyme citrate lyase and/or direct its action away from splitting the combined acetyl coenzyme A-oxaloacetate structure. The resultant drop of acetyl coenzyme A levels in the cytoplasm decreases fat synthesis. These inventors further believe that pregnane glycosides inhibit the action of malonyl coenzyme A in fat synthesis. Thus, caralluma glycosides provide two-fold action in decreasing fat synthesis: one by decreasing the formation of malonyl coenzyme A and the other by inhibiting the action of malonyl coenzyme A generated.

Decrease of malonyl coenzyme A levels shifts the malonyl coenzyme A-carnitine acyltransferase balance in favour increased fat breakdown as opposed to fat synthesis. Thus, under the effect of pregnane glycosides a body not only decreases fat synthesis but speeds up fat breakdown(fat metabolism). The effect is greater release of energy, that is an enhancement of BMR. The latter effect is more significant because the scope for decrease in fat synthesis is quite small in view of the fact that the amounts of fat synthesised by a body in a day is, in any case, a small quantity. The increased fat metabolism and the increase of BMR makes a subject feel more energetic unlike as in the case of the known appetite suppressants and, as will be observed from the further description hereinbelow, these together with other properties of the pregnane glycosides has a cascade effect on so many other body functions/conditions/ parameters and processes causing improvements thereof. Pregnane glycosides are thus energy and stamina enhancers and provide increased endurance. This mechanism of action of pregnane glycosides has been first observed by these inventors.

These inventors also found that pregnane glycosides also act on the hypothalamus to generate a feeling of satiety and well-being and reduce the feeling of hunger. This occurs without any side effects such as those associated with known appetite-suppressants. Pregnane glycosides also act in the liver to direct the lipids towards glycogen production. Increased glycogen level also contributes towards the reduction in the feeling of hunger felt by a subject.

This mechanism of the action of pregnane glycosides in relation to obesity has apparently not been known/presented before in the prior art. The description given hereinabove establishes the said effects of the pregnane glycosides in relation to the various symptoms/disorders/functions/parameters/conditions associated with obesity. The basis of said effects in relation to obesity is the appetite-reducing, the fat synthesis disruption, the fat metabolism increase and other properties of the pregnane glycosides. Specifically, the obesity and obesity related functions/parameters/conditions/disorders/ symptoms that are affected by pregnane glycosides are: weight, obesity, BMR, BMI, blood sugar, BP, blood lipids, appetite, lean body mass, waist, arm and hip circumferences, joints

The intake of caralluma glycosides by population groups that consume caralluma as a food may be anywhere upto 1500 mg. per day. Further, an intensive LD 50 safety pharmacological study conducted at the St John's Medical College and Hospital, Bangalore, India according to the OECD guidelines for testing of chemicals(Acute Oral Toxicity-Fixed Dose Method) showed no mortality in rats upon administration of a very high dose of 5 gms. per kg. body weight of caralluma fimbriata extract containing about 50% w/w of pregnane glycosides. No fatalities or adverse effects were noted. These data indicate that the toxicity limit if any of pregnane glycosides is quite high and may be well above 5000 mg. per day for humans. This also establishes that accidental overdoses of pregnane glycosides(or caralluma extracts) do not pose any risk.

The clinical trials programme done at the St. John's Medical College and Hospital, Bangalore, Inula was double-blind, controlled and randomised and followed the guidelines of the Indian Council of Medical Research, New Delhi, India with regard to methodology and ethical considerations and other factors. Sixty-two obese subjects were selected at random for the test, fifty of who completed the test, the rest having dropped out during the test. Half of the subjects who completed were on active medicine and the rest were on placebo.

Each subject was examined at the commencement of the trials and then at the end of the first and second months. The examination included anthropometric parameters of body weight, waist circumference, MAC, hip circumference, Fat%, BIA Fat% and lean% and a series of biochemical measurements including blood sugar lipid profile and others. The subjects were questioned about hunger level, urge to eat, fullness and thoughts on food and their responses recorded.

Both parametric(paired 't' test) and non-parametric tests such as Wilcoxon Signed Rank test(paired analysis) and Mann-Whitney test(for unpaired analysis) were used to look for significant changes between time points and between the groups. Both parametric and non-parametric tests gave similar results.

In groupwise analysis, the Wilcoxon-Signed Rank Test was used to check differences between time points separately in each group. The significant values were based on p < 0.0016. Paired 't' test analysis were used where differences were looked for in mean values between time points separately in each group. Significant differences were based on p<0.016(a value of 0.05 corrected with Bonferroni correction for three multiple comparisons for each analysis).

In the inter-group analysis, differences in change in each parameter were compared between the groups using Mann-Whitney test for independent comparisons. Significant differences were taken where p<0.05.

The overall conclusions from the trials are that statistically significant differences between time points were seen in the active group for the parameters of body weight, BMI, waist circumference, hip circumference, fat loss, blood pressure and hunger levels while blood sugar and lipid profile did not show any significant results.

The doses administered to the subjects consisted of an extract of caralluma fimbriata. The aerial parts of the plant were extracted with 30% v/v aq. ethanol. Resin removal was done with n-hexane solvent. This yielded the caralluma glycosides in aq. ethanol solution. This was concentrated and adsorbed on a suitable excipient. The material was then dried and filled in hard gelatine capsules. The dried, adsorbed material contained either about 25% or 50% w/w glycosides. Each capsule contained 500 mg. of the said excipient adsorbed extract containing either 25% or 50% w/w pregnane glycosides giving capsules of two strengths, single and double. The subjects took two capsules a day, one before each meal. The capsules given to the placebo group did not contain the extract. For some tests involving low value doses, capsules containing 250 mg. of said extract containing 25% and 50% w/w pregnane glycosides were used.

The adverse effects observed were GI(gastro-intestinal) in nature and were reported in both the groups, active and placebo. The effects were moderate acidity, mild constipation and mild to moderate flatulence and subsided within a week of commencement of trials. No adverse effects were noted in other systemic functions. No changes in ECG were observed. No sympathomimetic effects were found.

The American study by Dr. Ronald Lawrence and Dr. Suneeta Chaudhary at the Western Geriatric Research Institute, Los Angeles, California, USA was done on 26 randomly selected overweight patients of whom 19 were placed in the active group while 7 were on placebo. The trials were done over a 4-week period.

The subjects were taken from two active practices in the Los Angeles area and randomly assigned to the two groups. The age profile varied from 31 to 73. Two subjects dropped out during the trials leaving 24 to complete the test.

The following parameters were measured before and at the end of the tests: weight, height, hip and waist circumference and b.p. All the subjects were advised to pursue normal pattern of activity, exercise and food intake and not to alter their diets during the test.

The active group were given gelatine capsules containing an extract of Caralluma fimbrista. The capsules for the placebo did not contain the extract. The subjects were asked to take two capsules a day one prior to each meal. The active capsules contained 500 mg. of the extract each, said extract containing about 50% w/w of pregnane glycosides.

The extract incorporated in the capsules administered in the US study was prepared as follows. Aerial parts of Caralluma fimbriata were extracted with aq ethanol and then the extract was subjected to resin removal. The extract was then concentrated, adsorbed on a suitable excipient and the material dried and then incorporated in gelatine capsules.

The American study concludes that administration of Caralluma fimbriata extracts used in the weight reduction programme coupled with no change in daily activity pattern and diet of the subjects resulted in a statistically significant weight loss over a period of only four weeks. The study noted the lack of toxicity and the absence of any side effects. The study recommends further trials and states that there are few, if any over-the-counter natural substances which can produce such a weight reduction effect.

At the conclusion of the Indian study, the subjects expressed a voluntary desire to continue with the caralluma glycosides doses. The study was therefore extended and has already run over 12 months and is continuing. This has proved advantageous as the period of the earlier study was only two months. In the continuation study, the dosage has been increased to three capsules a day in a two-plus-one system. The increased dosage and the longer period have given some important insights that were not apparent earlier. Thus, while the earlier test did not show statistically significant effects as regards the blood sugar of the subjects, the link was clearly evident from the results of the extended study. The extended tests have established that administration of pregnane glycosides does lead to reduction of blood sugar levels, reduction of BP, reduction of serum cholesterol and reduction of LDL together with enhancement of HDL cholesterol in blood.

The said uses are described hereinbelow.

### 1. Obesity:

1.1 For subjects of either gender suffering from clinical obesity with an BMI of about 25 to 30, Type 2 diabetes or normal, normotensive or mild to moderats hypertension with no systemic dysfunction, the subject being preferably on controlled diet and/or moderate physical activity, otherwise no restriction. Main Dose: From about 250 to 500 mg. of caratuberside (CTB) per day over a period of 3 to 4 months followed by an optional maintenance dose of from about 125 to 250 mg. CTB per day for about six to eight months. Said maintenance dose may be taken over an extended period or indefinitely without any adverse effects as caralluma pregnane glycosides are good anti-oxidants, non-toxic and well-tolerated nutritional supplements.
1.2 For subjects of either gender suffering from clinical obesity with an BMI of about 30 to 50, Type 2 diabetes or normal, normotensive or mild to moderate hypertension with no systemic dysfunction, the subject being preferably on controlled diet and/or moderate physical activity, otherwise no restriction, a treatment dose double that provided above for the lower BMI category of subjects and optional maintenance dosage the same as for the lower BMI category is provided, all other parameters including the period for the doses being the same as for the lower BMI category..
1.3 An alternative schedule for the maintenance dose is to take the same for a period of about 4-5 months and then stop the same for a period of about 6 months. The maintenance dose may be again started at the end of the said 6 month period and continued for about three months. The maintenance course may be continued under this sequence of six and three months for an indefinite period. The water intake should be double of the normal during the treatment During the treatment one-half hour brisk walks in the morning and evening, and diet control, are advised.
   By about the fifth week, subjects start feeling the lessening of appetite and of the thoughts of food and simultaneously feel more energetic. Appetite is experienced by the subjects at appropriate times but is satisfied with lesser amounts of food. From this point onwards, weight loss also begins to become quite apparent. By the ninth week, the effects will be clearly apparent in weight loss, appetite reduction, reduction of waist, hip and arm circumferences and other parameters. The waist circumference would be down by atleast about 50 mm(two inches) and the weight by at least 3-4 kgs.
1.4 An alternative treatment for obesity provided by this invention comprises a mixture of PG and HCA.

Main dose: From about 120 to 240 mg. of CTB with from about 150 to 300 mg. of HCA per day over a period of about six months. Optional Maintenance dose: same as the main dose. Period: extended period to indefinitely.

### Garcinia extract may be used in place of HCA.

In the case of subjects where the gelatine of the capsules causes adverse GI reactions, the dose may be incorporated in a beverage and consumed in the liquid form. This applies to all of the treatments mentioned in this specification. This invention provides for a number of health-ensuring and nutracautical compositions containing PG and other components, the compositions being provided in both solid and beverage forms.

### 2. BMR: (not part of the invention)

2.1 For subjects undergoing moderate physical activity and desiring increase in BMR and energy, endurance and stamina levels.
   Main Dose: From about 250 to 500 mg. of CTB per day over a period of about 3 to 4 months. Optional maintenance dose: From about 125 to 250 mg. CTB per day over an extended period or indefinitely.
2.2 For subjects undertaking heavy physical activity such as sportspersons and desiring increase in BMR, energy, endurance and stamina levels:
   Main Dose: From about 500 to 1000 mg. CTB per day for a period of about 3 to 4 months.
   Optional maintenance dose: From about 250 to 500 mg. CTB per day for an extended period or indefinitely.
2.3 An alternative treatment for subjects desiring moderate increased BMR, stamina, energy and endurance levels:
   Main Dose: From about 90 to 150 mg, of CTB containing about 2 mg, to 15 mg. of saponin glycosides of Caralluma together with from about 100 to 200 mg. of Green tea catechins per day.
      Optional Maintenance dose: Same as the main dose. Period: extended to indefinite.
2.4 A yet another course of treatment for subjects desiring increase in BMR, energy, endurance and stamina:
   Main Dose: From about 100 to 200 mg. of CTB containing about 2 mg. to 15 mg. of saponin glycosides of caralluma together with from about 100 to 200 mg. of the catechins of green tea and from about 100 to 200 mg. of the withanolides of Ashwagandha per day.
   Period: About 6-9 months.
   Optional Maintenance dose: Same as the main dose Period: extended to indefinite.
   At the end of week two, the feeling of fatigue during exercise and workouts comes down and the subject will feel more energetic and capable of more exercise. At week four, male subjects on exercise programmes such as weight training will notice an upward trend in bicep circumference, chest circumference and increase in muscle sizes. Female subjects on weight training will see clear signs of loss of fat surrounding muscle groups. The treatment is suitable for housewives whose energy levels tend to sag after the morning round of work.

### 3. Lean Body Mass: (not part of the invention)

3.1 For subjects undertaking moderate physical activity and desiring increase in lean body mass: Main Dose: From about 250 to 500 mg. CTB per day over a period of about 3 to 4 months followed by an optional maintenance dose of from about 125 to 250 mg. CTB per day over an extended period or indefinitely.
3.2 For subjects undertaking heavy physical activity and desiring increase in lean body mass the main dose provided by the invention is from about 500 to 1000 mg. of CTB per day over a period of 3 to 4 months followed by an optional maintenance dose of from about 250 to 500 mg. CTB per day over an extended period or indefinitely.

### 4. Osteo-arthritis: (not part of the invention)

4.1 For subjects having early to middle stage osteo-arthritis of weight-bearing joints with mild to moderate radiological symptomatology and desiring relief from joint pains and inflammation. Main Dose: From about 250 to 500 mg. CTB per day over a period of about 4 to 5 months followed by an optional maintenance dose of from about 125 to 250 mg. CTB per day over an extended period or indefinitely.
4.2 For subjects suffering from severe osteo-arthritis of weight-bearing joints with mild to moderate radiological symptomatology and desiring relief from joint pains and inflammation:
   Main Dose: From about 500 to 1000 mg. CTB per day over a period of about 4 to 5 months followed by an optional maintenance dose of from about 250 to 500 mg. CTB per day over an extended period or indefinitely.
   Six alternative courses of treatment for subjects suffering from osteo-arthritis and/or desiring rejuvenation of the weight bearing joints and/or relief from joint pains are provided hereinbelow.
4.3 Main Dose: From about 120 to 300 mg. CTB together with from about 400 to 1000 mg. of Glucosamine sulphate per day for a period of about 6 to 8 months followed by an optional maintenance dose same as the main dose for a period of about 6 months or indefinitely.
4.4 Main Dose: From about 90 to 270 mg. of CTB together with from about 250 to 750 mg. of Glucosamine sulphate and from about 75 to 250 mg. of Rutin per day over a period of about 6 to 8 months followed by an optional maintenance dose same as the main dose for an extended period or indefinitely.
4.5 Main Dose: From about 120 to 300 mg. CTB together with from about 300 to 900 mg. Rutin per day over a period of about 6 to 8 months followed by an optional maintenance dose same as the main dose over an extended period or indefinitely. The maintenance dose may be adopted when the arthritic condition comes under control and the same may be taken in two parts, one morning and one evening.
4.6 Main Dose: From about 100 to 200 mg. CTB together with from about 600 to 750 mg. glucosamine sulphate per day to be taken in two parts. This dose is provided by the invention as a dietary supplement for subjects over forty years of age and desiring to prevent the onset of osteo-arthritis by the joints going into a degenerative process. This dose ensures adequate synovial fluid secretion sad rejuvenates the ligaments. In the hereinmentioned doses the bioflavonoid Rutin may be substituted by one of the other bioflavonoids and the glucosamine sulphate may be either as the sodium or potassium salt or other. The doses that exclude glucosamine sulphate are advised for subjects that have gastricacidity problem as glucosamine sulphate has an acidic reaction in the stomach. Period: Over an extended period or indefinitely.
4.7 Main dose: From about 120 to 300 mg. of CTB together with from about 300 to 900 mg. Rutin and from about 50 to 100 mg. of bamboo silica(70%)(or equivalent amount of another concentration) per day over a period of about six to eight months followed by an optional maintenance dose same as the main dose to be taken over an extended period or indefinitely. The main dose may be stopped and the maintenance dose adopted as soon as the arthritic condition is under control.
4.8 Main dose: From about 90 to 270 mg. of CTB together with from about 250 to 750 mg. of Glucosamine sulphate, from about 75 to 250 mg, of Rutin and from about 50 to 100 mg. of Bamboo silica(70%)(or equivalent amount of another concentration) per day over a period of about six to eight months followed by an optional maintenance dose same as the main dose for a period of about six months or indefinitely.

The pregnane glycosides in the abovementioned doses for osteo-arthritis provide the anti-inflammatory action and regenerative action. This is important as both glucosamine sulphate and chondroitin do not possess anti-inflammation properties. The subjects may be on NSAID therapy and/or physiotherapy treatment. Caralluma extracts are well-tolerated and exhibit no side effects on prolonged consumption. Preferably the doses may be taken after the meals.

At week three, gradual alleviation of pain occurs together with an increase in joint mobility and increased tolerance to physical stress. At week five, the subjects experience near-normaley in joint movements and cessation of pain, significant reduction in morning stiffiness, increased tolerance to exercise and quicker recovery from exercise. At week 13, further bone degradation ceases almost totally as established by radiological investigations.

Where combination main doses have been provided, the same may be discontinued when the arthritic condition comes under control. From that point, a maintenance dose of from about 250 to 300 mg of CTB per day may be adopted for a period of six to eight months. The said maintenance dose may be preferably taken in two stages after the two main meals of the day. For severe cases, the combination doses may preferably be continued indefinitely.

### 5. Blood Sugar: (not part of the invention)

5.1 The treatment provided by this invention for subjects having Type 2 diabetes and FBS(Fasting Blood Sugar) of about 120-150 mg./dL
   Main Dose: From about 500 to 1000 mg. CTB per day Period: prolonged to indefinite
5.2 For subjects having Type 2 diabetes and FBS exceeding about 150 mg/dL
   Main Dose: From about 1000 to 1500 mg. CTB per day over a prolonged period or indefinitely. The following two alternative courses of treatment are provided by the invention.
5.3 For subjects having Type 2 diabetes and/or desiring control/regulation of blood sugar:
   Main Dose: From about 100 to 250 mg. CTB together with from about 100 to 200 mg, of 4-hydroxy-iso-leucine(or as Fenugreek extract) per day, the PG containing about 2-3% bitters of the Caralluma species of plants. Period: 6 to 8 months.
   Optional maintenance dose same as the main dose. Period: over an extended period or indefinitely.
5.4 For subjects having Type 2 diabetes.
   Main Dose: From about 100 to 200 mg. of CTB together with from about 100 to 200 mg. of Coccinia extract containing about 10% terpenes, from about 100 to 200 mg. of Bitter gourd extract containing about 8% bitters, from about 100 to 200 mg. of Cinnamon extract containing about 15% polyphenols and from about 100 to 200 mg. Fenugreek extract containing about 40% 4-hydroxy-iso-leucine per day for a period of about 6-7 months followed by an optional maintenance dose, same as the main dose, preferably for an indefinite period.

Preferably the doses are taken 30 min. after meals. The dose may be taken in one stage or in two. At week two the subject would experience a reduction of about 10% in FBS and PPBS(Post-prandial blood sugar). At week four onwards, weight loss will be observed and also increased physical stamina. Blood sugar levels drop further. About 20% reduction in FBS and PPBS can be expected at this period. At week 16, reduction in giycosylated haemoglobin will be observed. Subjects may stop their main dose after the stipulated period for a period of about 6-8 months. They may then revive the main dose for about 2-3 months after a gap of about 2-3 months. This sequence may be continued indefinitely. For subjects over forty years of age, it is preferable that the said sequence is continued indefinitely. For subjects over fifty, preferably the main dose should be continued indefinitely. Regular exercise in the form of a brisk walk of 45 min. morning and evening is advised and it is suggested that subjects avoid refined carbohydrate foods.

### 6. Blood Pressure: (not part of the invention)

6.1 For subjects suffering from mild to moderate hypertension the main dose provided by the invention comprises from about 250 to 500 mg. of CTB per day to be taken over a period of about 3 to 4 months followed by an optional maintenance dose of from about 125 to 250 mg. of CTB per day for an extended period or indefinitely.
6.2 For subjects having severe hypertension the treatment provided by the invention comprises: Main Dose: From about 500 to 1000 mg. of CTB per day for a period of about 3 to 4 months followed by a maintenance dose of from about 250 to 500 mg. of CTB per day for an extended period or indefinitely.
6.3 An alternative course is provided by the invention for subjects having mild to moderate hypertension wherein the main dose is from about 125 to 250 mg. of CTB together with from about 125 to 250 mg. of HCA(Garcinia extract) per day to be taken over an indefinite period.
6.4 A still another alternative course of treatment and management for this condition provided by the invention comprises a main dose of from about 125 to 250 mg. of CTB together with from about 125 to 250 mg. of Commiphora Mukul extract containing about 3% gugulsterones per day to be taken over an indefinite period.

The tests have established that pregnane glycoside(s) reduce blood pressure, both systolic and diastolic. Another related effect is that of reducing serum LDL(Low density lipoprotein) and enhancing the HDL (High density lipoprotein). The dose may be taken in one stage or two and is preferably taken after meals. At week three, increased energy and exercise endurance were observed. This brings down the LDL levels. Physical training is advised during the treatment. At week five, increase in the HDL level and further increases in energy and endurance are observed.

### 7. Appetite Reduction: (for illustrative purpose only)

7.1 For subjects desiring mild to moderate reduction in appetite, this invention provides: Main Dose: from about 250 to 500 mg. of CTB per day over a prolonged period or indefinitely.
7.2 For subjects desiring heavy reduction in appetite: Main Dose: From about 500 to 1000 mg. of CTB per day over a prolonged period or indefinitely.
7.3 This invention provides an alternative course comprising pregnane glycosides and HCA. Main dose: From about 120 to 240 mg. of CTB with from about 3 s0 to 300 mg. of HCA per day over a period of about six months. Optional Maintenance dose: same as the main dose. Period: extended period to indefinitely.

### Garcinia extract may be used in place of HCA.

### 8. Weight reduction: (for illustrative purpose only)

8.1 For subjects desiring slow/gradual reduction in weight: Main Dose: From about 250 to 500 mg. of CTB per day over a period of about 3 to 4 months followed by an optional maintenance dose of from about 125 to 250 mg. CTB per day taken over an extended period or indefinitely.
8.2 For subjects desiring rapid reduction in weight Main Dose: From about 500 to 1000 mg. CTB per day over a period of about 3 to 4 months followed by an optional maintenance dose of from about 250 to 500 mg. CTB per day over an extended period or indefinitely.
8.3 This invention provides an alternate course comprising pregnane glycosides and HCA. Main dose: From about 120 to 240 mg. of CTB with from about 150 to 300 mg. of HCA per day over a period of about six months. Optional Maintenance dose: same as the main dose. Period: extended period to indefinitely.

### Garcinia extract may be used in place of HCA.

### 9. Waist, arm and hip circumference: (for illustrative purpose only)

9.1 For subjects desiring reduction in waist, hip and arm circumferences: Main Dose: From about 250 to 500 mg. of CTB per day over a period of about 3 to 4 months followed by an optional maintenance dose of from about 125 to 250 mg. CTB per day ever an extended period or indefinitely.
9.2 This invention provides an alternative course comprising pregnane glycosides and HCA. Main dose: From about 120 to 240 mg. of CTB from about 150 to 300 mg. of HCA per day over a period of about six months. Optional Maintenance dose: same as the main dose. Period: extended period to indefinitely.

### Garcinia extract may be used in place of HCA.

### 10. Migraine: (not part of the invention)

10.1 For a subject desiring relief from migraine: Main Dose: From about 500 to 1000 mg. of CTB per day over a period of about 3 to 4 months, said dose to be doubled during the periods of attack. The daily dose may be preferably taken in two parts one after each meal. At week five, the subject will notice decreased frequency of attacks. The pregnane glycosides exerts its anti-inflammatory properties and also restores elasticity to the capillaries. The anti-depressant property of pregnane glycosides gives a psychological boost to the subject. His confidence level increases due to increased secretion of serotonin. At week nine, the subject experience significantly reduced frequency of attacks and increased tolerance to physical stress. At week thirteen, the frequency of attacks can be expected to be down to less than 10%. A maintenance dose of from about 125 to 250 mg. of CTB per day may preferably be continued indefinitely

### 11. Clinical depression: (not part of the invention)

11.1 For a subject suffering from mild clinical depression: Main Dose: From about 125 to 250 mg. of CTB per day to be taken till the ceasing of the symptoms.
11.2 For subjects suffering from severe clinical depression: Main Dose: From about 250 to 1000 mg. CTB per day to be taken till the ceasing of the symptoms.
11.3 An alternative course provided by the invention for a subject suffering from clinical depression or desiring mood elevation comprises main dose of from about 250 to 500 mg. CTB per day together with from about 100 to 200 mg. of the withanolides of Ashwagandha over a period of about 12 to 18 months followed by an optional maintenance dose of from about 125 to 250 mg. of CTB per day together with from about 50 to 100 mg. of the withanolides of Ashwagandha over an extended period or indefinitely. Depressions are associated with abnormal levels of serotonin reuptake. Pregnane glycosides cause increases in serotonin levels without the adverse effects such as serotonin intoxication of known antidepressants such as the SSRI's. With pregnane glycosides, subjects experience increased feeling of well-being, increased energy levels, increase tolerance to stress and a general improvement in mood *inter alia* through its effect on serotonin levels. Adverse effects, if any, are of a milder form than with said SSRI's. The subjects begin to experience increased energy levels, reduced fatigue and tiredness and general well-being at week three. At week four, there is further all-round improvement in the abovementioned parameters and the subjects experience increased social interests.

### 12. Sexual dysfunction: (not part of the invention)

12.1 For a subject suffering from primary impotence and/or decreased libido and/or desiring increased libido and sexual drive, power and stamina: Main Dose: From about 500 to 1000 mg. of CTB per day over the period of existence of dysfunction and decreased libido or as desired by the subject.
12.2 An alternative course provided by the invention for a subject suffering from sexual dysfunction such as primary impotency and/or reduced libido or desiring increased sex drive, power and stamina: Main Dose: From about 250 to 500 mg. of CTB per day together with from about 100 to 200 mg. of the withanolides of Ashwagandha over a period of about 6 to 12 months followed by an optional maintenance dose of from about 125 to 250 mg. of CTB per day together with from about 50 to 100 mg. of the withanolides of Ashwagandha as long as necessary or as long as increase in sex power is desired.
12.3 A yet another alternative course provided by the invention for a subject suffering from sexual dysfunction such as primary impotence and/or loss of, or reduced libido and/or desiring increased sex power stamina and drive comprises:
   Main Dose: From about 250 to 500 mg. of CTB per day together with from about 100 to 200 mg. of Shilajith over a period of about 6 to 12 months followed by an optional maintenance dose of about 125-250 mg. CTB per day together with about 50-100 mg. of Shilajith for as long as necessary or as long as increased sex drive is desired.
12.4 A yet another course of treatment provided by the invention for a subject suffering from sexual dysfunction such as primary impotence and/or loss of, or reduced libido and/or desiring increased sex power/drive and stamina. Main dose: From about 250 to 500 mg. of CTB per day together with from about 250-500 mg. of Fenugreek extract containing about 50% Protodioscin to be taken over a period of about 3-4 months followed by an optional maintenance dose of from about 125 to 250 mg. of CTB together with from about 125 to 250 mg. of said fenugreek extract per day for as long as necessary or as long as increase sex drive is desired.

The effects ot pregnane glycosides relevant to its application in treatment of sexual dysfunction are: increase in energy, endurance and stamina, beneficial effect on capillaries and through that on circulation, feeling of increased well-being, increased tolerance to stress, elevation of mood and others. Together these effects appear to produce increased blood flow in the region of the reproductive organs and cause resurgence of sexual interest and increase of libido which has been clearly established by the tests/trials. Caralluma extracts also appear to be beneficial for women in the post-menopausal phase when they experienced reduced sexual desire, painful intercourse, diminished sexual responsiveness, difficulty in achieving orgasm and decreased genital sensation. Pregnane glycosides do not have the cardio-vascular and other side effects exhibited by the known compounds for treatment of sexual dysfunction, such as, for example, the PDE5 inhibitors.

### 13. Cognitive and memory function: (not part of the invention)

13.1 For a subject suffering from diminished cognitive and memory function or desiring increase thereof: Main Dose: From about 250 to 500 mg. of CTB per day over a period of about 3 to 4 months.
   Caralluma extracts have a beneficial effect on neurotransmitter levels and functioning and, in particular, on serotonin levels. It appears that the multiple effects of pregnane glycosides on a subject has a cascade type effect causing an all-round improvement in body functions including cognitive and memory function, memory recall and retention and speed of retrieval. This has been established by said tests/trials. The dose may be taken in a single stage ortwo and is preferably taken after meals.

### 14. Aging syndrome: (not part of the invention)

14.1 For subjects desiring treatment/management of the aging syndrome, this invention provides for a main dose of from about 250 to 500 mg. of CTB per day over a prolonged to indefinite period.
14.2 An alternative course provided by the invention comprises a dose of from about 100-200 mg. of CTB per day together with from about 100 to 200 mg. of the catechins of green tea and from about 100 to 200 mg. of the saponin glycosides of caralluma followed by an optional maintenance dose of from about 100 to 200 mg. of CTB per day together with from about 50 to 100 mg. of said catechins and from about 120 to 300 mg. of said saponin glycosides over an indefinite period.
14.3 This invention also provides a dietary supplement for fighting the aging syndrome comprising a dose of from about 100 to 200 mg. of CTB per day together with from about 250 to 350 mg. of Green Tea extract containing about 40% catechins to be taken indefinitely.
14.4 A yet another composition provided by this invention for a subject desiring reduction in the aging syndrome comprises a main dose of from about 50 to 100 mg. of CTB per day together with from about 100 to 150 mg. cf Zinc monomethionine, from about 150 to 250 mg. of Citrus bioflavonoids and selenium chelate equivalent to from about 2 to 8mg, of selenium to be taken over an indefinite period.

The properties of pregnane glycosides relevant to its application as an anti-oxidant and in combating aging are: i. Reduction of fat ii. Increase of energy levels and stamina levels iii. Increased physical activity iv. lowered stress and mood elevation v, increased jointmobility vi. increasing capillary elasticity and others. These benefits are obtained with substantially nil side effects.

### 15. Loss of Hearing: (not part of the invention)

15.1 For a subject suffering from hearing loss: Main Dose: From about 250 to 500 mg. of CTB per day over a period of about 3 to 4 months followed by an optional maintenance dose of from about 125 to 250 mg. of CTB per day taken over an extended period or indefinitely.

### 16. Circulation disorder: (not part of the invention)

16.1 For a subject suffering from a circulation disorder: Main Dose: From about 500 to 1000 mg. of CTB per day over a period of about 3 to 4 months followed by an optional maintenance dose of from about 250 to 500 mg. per day of CTB over an indefinite period. The invention provides for the maintenance dose to be taken indefinitely in case of severe circulation disorder and where the disorder is mild and the maintenance dose is not adopted then the main dose should be commenced upon re-occurrence of the disorder/symptom. The main dose must be revived if the circulation problem re-occurs and in cases of severe circulation problems the main dose should preferably be continued indefinitely.

### 17. Capillary Degeneration: (not part of the invention)

17.1For a subject desiring restoration or maintenance of capillary elasticity: Main Dose: From about 500 to 1000 mg. of CTB per day for a period of about 3 to 4 months followed by a maintenance dose of from about 250 to 500 mg of CTB per day to be taken over an indefinite period.

It has been observed that caralluma extracts enhance/restore capillary elasticity. Together with the other effects of pregnane glycosides, the subject experiences an all-round improvement of body functions including hearing, sexual function, skin health, mental functions, circulation and others. The dosage may be taken in one stage or two and is preferably taken in two stages after the two main meals of the day.

### 18. Skin Nourishment: (not part of the invention)

18.1 For a subject desiring skin nourishment and skin elasticity and health: Main Dose: From about 50 to 100 mg. of CTB per day together with from about 100 to 150 mg. of Zinc monomethiomne, from about 150 to 250 mg. of Citrus bioflavonoids and from about 2 to 8 mg. of selenium as selenium chelate or other compound, to be taken over an extended period or indefinitely.

### 19. Menopausal syndrome: (not part of the invention)

19.1 For a subject suffering from the menopausal syndrome and desiring alleviation from hot flushes and menopausal distress: Main Dose: From about 50 to 100 mg. of CTB per day together with from about 100 to 150 mg. of Zinc monomethionine, from about 150 to 200 mg. of Citrus bioflavonoids and from about 6 to 10 mg. of selenium as selenium chelate or other compound to be taken as long as necessary.
   Four further courses of treatment and management for a subject suffering from menopausal syndrome are indicated hereinbelow.
19.2 Main dose: From about 50 to 100 mg. of CTB together with from about 100-200 mg. Liquorice extract containing about 5% Triphytoestrogens per day to be taken as long as necessary and as long as symptoms last.
19.3 Main dose: From about 50 to 100 mg. of CTB together with from about 100 to 200 mg. of Red clover extract containing about 8% isoflavones per day to be taken for as long as necessary and as long as symptoms last.
19.4 Main Dose: From about 50 to 100 mg. of CTB together with from about 100 to 200 mg. of Hops flower extract containing about 5% triphytoestrogens per day to be taken for as long as necessary and as long as symptoms last.
19.5 Main dose: From about 50 to 100 mg. of CTB together with from about 100 to 200 mg. of Pomegranate extract containing about 10% Ellagic acid per day. Period: As long as the symptoms and as long as necessary.

### 20. Cancer prevention/protection: (not part of the invention)

20.1 For a subject desiring the neutralisation of carcinogens and protection against cancer: Main Dose: From about 50 to 100 mg. of CTB per day together with from about 100 to 150 mg. of Zinc monomethionine, from about 150 to 200 mg. of Citrus bioflavonoids and from about 2 to 8 mg. of selenium as selenium chelate or other compound to be taken indefinitely.

### 21. Cholesterol: (not part of the invention)

21.1 For a subject desiring reduction of total blood cholesterol: Main Dose: From about 125 to 500 mg. of CTB per day together with from about 150 to 250 mg. of Hibiscus Subdarifa extract containing about 25% polyphenols and from about 100 to 200 mg. of Commiphora Mukulextract containing about 3% gugulsterones to be taken for an indefinite period.
21.2 An alternative composition provided by the invention for a subject desiring reduction in blood cholesterol: Main Dose: From about 120 to 240 mg. of CTB per day together with from about 150 to 300 mg. of HCA over a period of about six months followed by an optional maintenance dose same as the main dose over an extended period or indefinitely preferably over an indefinite period.
21.3 A yet another alternative composition provided by the invention for a subject desiring reduction in blood cholesterol: Main Dose: From about 90 to 150 mg. of CTB containing about 2-10% saponin glycosides, together with from about 100 to 200 mg. of the catechins of green tea per day over an extended period or indefinitely.

### 22. BMI: (not part of the invention)

For a subject desirous of reducing BMI, the invention provides for the following courses of treatment and management.
22.1 For subjects of either gender having a BMI of about 25 to 30, Type 2 diabetes or normal, nonnotensive or mild to moderate hypertension with no systemic dysfunction, the subject being preferably on controlled diet and/or moderate physical activity, otherwise no restriction.
   Main Dose: From about 250 to 500 mg. of caratuberside(CTB) per day over a period of 3 to 4 months followed by an optional maintenance dose of from about 125 to 250 mg. CTB per day for about six to eight months. Said maintenance dose may be taken over an extended period or indefinitely without any adverse effects as caralluma pregnane glycosides are good anti-oxidants, non-toxic and well-tolerated nutritional supplements.
22.2 For subjects of either gender having a BMI of about:30 to 50, Type 2 diabetes or normal, normotensive or mild to moderate hypertension with no systemic dysfunction, the subject being preferable on controlled diet and/or moderate physical activity, otherwise no restriction, a treatment dose double that provided above for the lower BMI category of subjects and optional maintenance dosage the same as for the lower BMI category is provided, all other parameters including the period for the doses being the same as for the lower BMI category.
22.3 An alternative schedule for the maintenance dose is to take the same for a period of about 4-5 months and then stop the same for a period of about 6 months. The maintenance dose may be again started at the end of the said 6 month period and continued for about three months. The maintenance course may be continued under this sequence of six and three months for an indefinite period. The water intake should be double of the normal during the treatment During the treatment one-half hour brisk walks morning and evening and diet control are advised.
   By about the fifth week, subjects start feeling the lessening of appetite and of the thoughts of food and simultaneously feel more energetic. Appetite is experienced by the subjects at appropriate times but is satisfied with lesser amounts of food. From this point onwards, lowering of the BMI(weight loss) also begins to become quite apparent
22.4 An alternative composition of this invention for increase of BMI comprises: Main dose: From about 120 to 240 mg. of CTB with from 150 to 300 mg. of HCA per day over a period of about six months. Optional Maintenance dose: same as the main dose. Period: extended period to indefinitely.

### Garcinia extract may be used in place of HCA.

### 23. Anti-oxidation: (not part of the invention)

23.1 This invention provides for an anti-oxidation course for subjects desiring treatment/ management of the aging syndrome and/or for general fitness and health, comprising a main dose of from from about 250 to 500 mg. of CTB per day over a prolonged to indefinite period.
23.2 An alternative anti-oxidation course provided by the invention comprises a dose of from about 100 to 200 mg. of CTB per day together with from about 100 to 200 mg. of the catechins of green tea and from about 100 to 200 mg. of the saponin glycosides of caralluma followed by an optional maintenance dose of from about 100 to 200 mg. of CTB per day together with from about 50 to 100 mg. of said catechins and from about 120 to 300 mg. of said saponin glycosides over an indefinite period.
23.3 This invention also provides an anti-oxidant dietary supplement for fighting the aging syndrome and/or provide general health and fitness comprising a dose of from about 100 to 200 mg. of CTB per day together with from about 250 to 350 mg. of Green Tea extract containing about 40% catechins to be taken indefinitely.
23.4 A yet another composition course provided by this invention for a subject desiring reduction is the aging syndrome and/or ensuring general health and fitness comprises a main dose of from about 50 to 100 mg. of CTB per day together with from about 100 to 150 mg. of Zinc monomethionine, from about 150 to 250 mg. of Citrus bioflavonoids and selenium chelate equivalent to from about 2 to 8 mg. of selenium to be taken over an indefinite period.

The properties of pregnane glycosides relevant to its application as an anti-oxidant and in combating aging are: i. Reduction of fat ii. Increase of energy levels and stamina levels iii. Increased physical activity iv. lowered stress and mood elevation and v. increased joint mobility vi. improved circulation and others. These benefits are obtained with substantially nil side effects.

In all the treatments described hereinabove, the dosages may be taken before, during or after meals. However, where the objective is to provide relief from one or more of the obesity-related symptoms the dosages may be preferably taken one-half to one hour before meals. A daily dose may be taken at one time or in two stages. If taken at one time, it may be preferably taken before, during or after the main meal of the day.

The term 'indefinitely' used in relation to said treatment and maintenance doses is intended to mean that the doses are to be taken substantially lifelong in a continuous mode wherein the doses are taken daily without a break. The scope of the said term 'indefinitely' is intended to include the option of taking the maintenance doses in a periodical(sequential) arrangement(mode) comprising taking the doses periodically over periods of about two to seven months with the gaps also extending to about two to seven months.

In the applications related to obesity-related symptoms, the sequential formula for maintenance doses described hereinabove is recommended.

## Claims

1. Pregnane glycosides in the form of extracts of the caralluma species of plants for use in the treatment of obesity, wherein said treatment comprises the administration of a daily main dose thereof to a subject over a period of time of 3 to 4 month, the pregnane glycoside content of said main dose being specified by the molecularly equivalent amount of caratuberside therein and is from 250 mg to 500 mg caratuberside in case the BMI of the subject is from 25 to 30 or 500 mg to 1000 mg caratuberside in case the BMI of the subject is from 30 to 50.

2. Pregnane glycosides for use in the treatment of obesity according to claim 1, wherein the main dose is followed by a daily maintenance dose thereof, the pregnane glycoside content of said maintenance dose being specified by the molecularly equivalent amount of caratuberside therein and is from 125 mg to 250 mg caratuberside in case the BMI of the subject ranges from 25 to 50 and is to be taken for 6 to 8 month or indefinitely.

3. Pregnane glycosides for use according to the claims 1 to 2, wherein the pregnane glycosides are in the form of an aqueous ethanolic extract with a pregnane glycoside content of either from 5 % w/w to 15% w/w or exceeding 15% w/w.

4. Pregnane glycosides for use according to the claims 1 to 3, wherein the pregnane glycosides are in the adsorbed form over an excipient with a pregnane glycoside content of either from 25% w/w to 30% w/w or exceeding 30% w/w.

5. Pregnane glycosides for use according to claim 4, wherein the excipient is either Malto Dextrin or Magnesium Carbonate.

6. Pregnane glycosides for use according to the claims 1 to 5, wherein the pregnane glycosides are in the form of pharmaceutically accepted salts and/or on pharmaceutically accepted carriers.

## Patentansprüche

1. Pregnanglycoside in Form von Extrakten der Caralluma-Pflanzengattung zur Verwendung bei der Behandlung von Fettleibigkeit, wobei die Behandlung die Verabreichung einer täglichen Hauptdosis von diesen an einen Patienten über einen Zeitraum von 3 bis 4 Monaten umfasst, wobei der Gehalt an Pregnanglycosid der Hauptdosis durch die molekular äquivalente Menge an Caratubersid darin spezifiziert ist und 250 mg bis 500 mg Caratubersid bei einem BMI des Patienten von 25 bis 30 beträgt oder 500 mg bis 1000 mg Caratubersid bei einem BMI des Patienten von 30 bis 50 beträgt.

2. Pregnanglycoside zur Verwendung bei der Behandlung von Fettleibigkeit nach Anspruch 1,
wobei auf die Hauptdosis eine tägliche Aufrechterhaltungsdosis davon folgt, wobei der Gehalt an Pregnanglycosid der Aufrechterhaltungsdosis durch die molekular äquivalente Menge an Caratubersid darin spezifiziert ist und 125 mg bis 250 mg Caratubersid bei einem BMI des Patienten von 25 bis 50 beträgt und 6 bis 8 Monate oder für eine unbestimmte Zeit eingenommen werden muss.

3. Pregnanglycoside zur Verwendung gemäß den Ansprüchen 1 bis 2,
wobei die Pregnanglycoside in Form eines wässrigen Ethanolextraktes mit einem Gehalt an Pregnanglycosid von entweder 5 Gew.-% bis 15 Gew.-% oder von über 15 Gew.-% vorliegt.

4. Pregnanglycoside zur Verwendung gemäß den Ansprüchen 1 bis 3,
wobei die Pregnanglycoside in adsorbierter Form über ein Vehikel mit einem Gehalt an Pregnanglycosid von entweder 25 Gew.-% bis 30 Gew.-% oder von über 30 Gew.-% vorliegt.

5. Pregnanglycoside zur Verwendung gemäß Anspruch 4,
wobei das Vehikel entweder Maltodextrin oder Magnesiumcarbonat ist.

6. Pregnanglycoside zur Verwendung gemäß den Ansprüchen 1 bis 5,
wobei die Pregnanglycoside in Form von pharmazeutisch verträglichen Salzen und/oder pharmazeutisch verträglichen Trägern vorliegen.

## Revendications

1. Glycosides de prégnane sous forme d'extraits de l'espèce de plantes caralluma pour une utilisation dans le traitement de l'obésité, où ledit traitement comprend l'administration d'une dose principale de celle-ci quotidienne à un sujet sur une période de temps de 3 à 4 mois, la teneur en glycosides de prégnane de ladite dose principale étant spécifiée par la quantité équivalente au niveau moléculaire de caratuberside à l'intérieur et est de 250 à 500 mg de caratuberside dans le cas où l'IMC du sujet est de 25 à 30 ou de 500 à 1 000 mg de caratuberside dans le cas où l'IMC du sujet et de 30 à 50.

2. Glycosides de prégnane pour une utilisation dans le traitement de l'obésité selon la revendication 1,
où la dose principale est suivie par une dose de maintenance quotidienne de celle-ci, la teneur en glycosides de prégnane de ladite dose de maintenance étant spécifiée par la quantité équivalente au niveau moléculaire de caratuberside à l'intérieur et est de 125 à 250 mg de caratuberside dans le cas où l'IMC du sujet se situe dans la plage allant de 25 à 50 et doit être prise durant 6 à 8 mois ou indéfiniment.

3. Glycosides de prégnane pour une utilisation selon les revendications 1 à 2,
dans laquelle les glycosides de prégnane sont sous la forme d'un extrait éthanolique aqueux avec une teneur en glycosides de prégnane de 5 à 15 % p/p ou dépassant 15 % p/p.

4. Glycosides de prégnane pour une utilisation selon les revendications 1 à 3,
dans laquelle les glycosides de prégnane sont sous forme adsorbée sur un excipient avec une teneur en glycosides de prégnane de 25 à 30 % p/p ou dépassant 30 % p/p.

5. Glycosides de prégnane pour une utilisation selon la revendication 4,
dans laquelle l'excipient est ou la maltodextrine ou le carbonate de magnésium.

6. Glycosides de prégnane pour une utilisation selon les revendications 1 à 5,
dans laquelle les glycosides de prégnane sont sous la forme de sels pharmaceutiquement acceptés et/ou sur des véhicules pharmaceutiquement acceptés.
